# EUROPEAN PATENT SPECIFICATION

(11) **EP 2 131 764 B1**
(45) Date of publication and mention of the grant of the patent: **28.03.2012**
(21) Application number: 08713141.3
(22) Date of filing: 15.01.2008
(51) Int. Cl.: A61F 6/08

(54) **CONCEPTION CAP**
INSEMINATIONSKAPPE
CAPUCHON DE CONCEPTION

(30) Priority: 28.03.2007 US 692511
(43) Date of publication of application: 16.12.2009
(73) Proprietor: Melrock LTD., Jersey Channel Islands JE2 3JW (GB)
(72) Inventor: LA VEAN, Michael, Saranac, Michigan 48881 (US)
(74) Representative: Gleiss, Alf-Olav
(86) International application number: PCT/US2008/000505
(87) International publication number: WO 2008/118249

(56) References cited:
- WO-A1-99/37259
- WO-A1-2006/058409
- US-A- 4 703 752
- US-A- 4 895 170
- US-A- 4 961 436
- US-A- 5 207 232
- US-A- 5 857 959

## Description

The present disclosure relates generally to a conception cap used to concentrate semen and effect fertilization. The present disclosure also generally relates to a method of conception utilizing the cap. Furthermore, the present disclosure relates to a conception cap that may be implanted by the user in the comfort of her own home and which does not limit normal physical activity while in use.
The document WO-A-99/37259 represents the closest prior art.

### INTRODUCTION

The statements in this section merely provide introductory information related to the present disclosure and may not constitute prior art.

Medical devices intended to be inserted into the vagina and secured to the cervix are known for use as contraceptive barriers. One particular contraceptive device, the cervical cap, may be placed over the cervix to prevent semen from entering the cervical canal. The cervical cap may be held in place by a suction grip or surface viscosity on the moist cervical surface. Insofar as known devices are intended for the prevention of pregnancy, latex has proven to be a suitable material. Latex, however, may result in semen damage. Thus, a latex cap should not be used for delivery of semen.

To a more limited extent, it is known in the pertinent art to provide a cervical cap to position a quantity of semen in proximity to the cervix for purposes of facilitating impregnation. In this regard, U.S. Patent No. 5,857,959 illustrates and describes a conception kit developed by the inventors of the present disclosure. The kit generally includes a conception cap comprising a thin, form-assuming, flexible dome, an annular rim, and a pair of gripping flanges along an inner surface of the rim for positioning and securing the cap over the cervix. The cap concentrates semen on the cervical os to effect fertilization. While the conception kit shown and described in U.S. Patent No. 5,857,959 has proven to be extremely successful in promoting pregnancy, continued improvement in the pertinent art remains desirable.

Some of the primary factors contributing to a decline in fertility include low semen counts, problems with semen motility, tilted cervix, and a hostile vaginal environment due to infection or other chronic conditions. The present disclosure provides an improved conception cap and related method for even more effectively concentrating semen for successful fertilization, thereby even better overcoming the various factors associated with fertility decline, including, but not limited to, the aforementioned factors. The conception cap of the present disclosure may be made of an implantable material such as a silicone-based material, and may be positioned and secured over the cervix while containing semen to facilitate conception. Moreover, the construction of the conception cap allows a woman to increase the likelihood of conception within the comfort, convenience and privacy of her own home, and does not limit normal physical activity.

### SUMMARY

The present disclosure generally relates to a conception cap that is positioned over the cervix to increase the chances of successful fertilization. A dome of the conception cap is designed to contain semen and, upon securement, properly position a higher concentration of semen in proximity to the cervical os. The conception cap is easily positioned, comfortable to use, and easily removed.

According to one particular aspect, the present teachings provide a conception cap that may be positioned over a cervix to concentrate semen and promote fertilization. The conception cap may include an annular rim and a dome. The dome may extend from the annular rim and define a receptacle area. The dome may have a closed tip, a base portion and a sidewall extending between the closed tip and the base portion. The sidewall may be collapsible. The annular rim and the dome are formed of a material suitable for use in the vagina.

According to another particular aspect, the present teachings similarly provide a conception cap for positioning over a cervix to concentrate semen and promote fertilization. The conception cap may include an annular rim and a dome. The dome may extend from the annular rim and define a receptacle area. The dome may have a closed tip, a base portion and a sidewall extending between the closed tip and the base portion. The conception cap may further include at least three thin, gripping flanges projecting radially inwardly from the annular rim. Adjacent flanges may be spaced apart by a notch to permit the flanges to deflect towards the closed tip of the dome during insertion of the cap, and to effectively grip and hold the cap over the cervix. The dome and the annular rim may be formed of a material suitable for use in the vagina.

According to yet another particular aspect, the present teachings again provide a conception cap for positioning over a cervix to concentrate semen and promote fertilization. The conception cap may include an annular rim, a dome and a handle. The annular rim may generally define a plane. The dome may extend from the annular rim and define a receptacle area. The dome may include a closed tip, a base portion, and a sidewall extending between the closed tip and the base portion. The handle may extend from the annular rim at an angle to the plane defined by the annular rim. The dome, annular rim, and handle may be formed of a material suitable for use in the vagina.

Further areas of applicability of the present teachings will become apparent from the description and appended claims provided hereinafter. It should be understood that the detailed description and specific examples, while indicating the various examples of the present teachings, are intended for purposes of illustration only and are not intended to limit the scope of the teachings.

### DRAWINGS

The present teachings will become more fully understood from the detailed description, the appended claims and the following drawings.

Figure 1 is a perspective view of a conception cap in accordance with the present teachings.

Figure 2 is a side view of a conception cap in accordance with the present teachings.

Figure 3 is a top view of the conception cap in accordance with present teachings.

Figure 4 is a cross-sectional view taken along line 4-4 of Figure 3.

Figure 5 is a side view of a conception cap according to the present teachings similar to Figure 2, the dome of the conception cap illustrated as it may collapse during use.

### DESCRIPTION OF VARIOUS ASPECTS

The following description is merely exemplary in nature and is not intended to limit the present disclosure. It will be understood that corresponding reference numerals indicate like or corresponding parts and features throughout the drawings. The description and any specific examples, while indicating embodiments of the present disclosure, are intended for purposes of illustration only and are not intended to limit the scope of the present disclosure. Moreover, recitation of embodiments having stated features is not intended to exclude other embodiments having additional features, or other embodiments incorporating different combinations of the stated features.

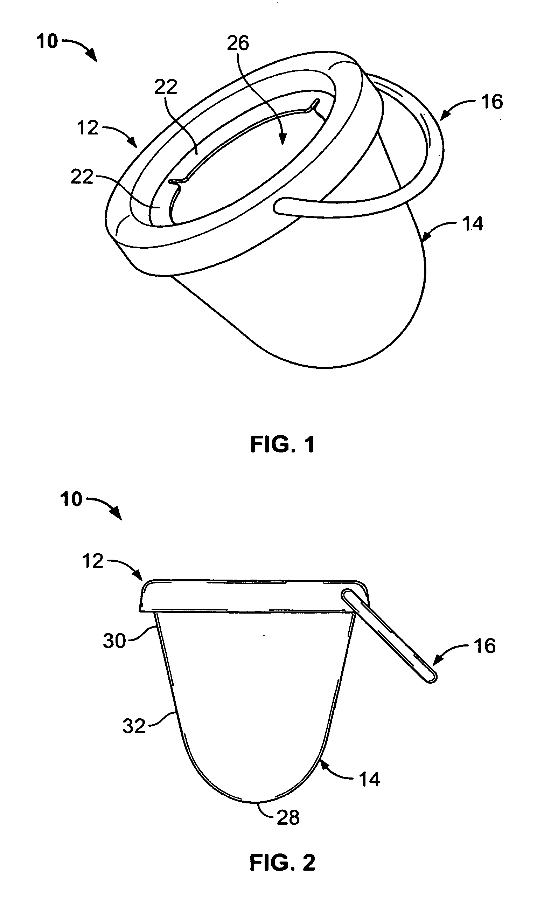

Referring generally to Figures 1-5 of the drawings, a conception cap in accordance with the present teachings is illustrated and generally identified by numeral 10. The conception cap 10 may be utilized in connection with a conception kit for purposes of promoting pregnancy. One suitable conception kit is shown and described in U.S. Patent No. 5,857,959 . It will be understood, however, that the various teachings of the present disclosure may be employed with other conception kits within the scope of the present invention.

The conception cap 10 may generally include an annular rim 12, a dome 14, and a handle 16. The annular rim 12 has an inner surface 18 and an outer surface 20. One or more cervical engagement members 22 may radially extend inward from the inner surface 18 of the annular rim 12. The one or more engagement members 22 may include a plurality of flanges 22. The plurality may include two or more flanges 22 extending through approximately 120 degrees or less. As illustrated in the drawings, the plurality may include three flanges. In other applications, the plurality may include four or more flanges 22. The flanges 22 may be thin in an axial direction and internally formed with the annular rim 12. Adjacent flanges 22 may be spaced apart by a notch 24.

The flanges 22 and cooperating notches 24 may effectively grip and hold the conception cap 10 over the cervix in order to concentrate the semen at the os of the cervix and to successfully effect fertilization. The flanges 22 and notch 24 essentially provide the effect of a Chinese finger puzzle by gripping the side walls of the cervix and holding the conception cap 10 when the circumference of the annular rim 12 is fitted around the cervix and slightly expands. The conception cap 10 is fixed in place by the use of the flanges 22, rather than merely by suction or surface viscosity.

Because the individual flanges 22 extend through approximately 120 degrees or less, bunching up of the deflected flanges 22 is avoided. These flanges 22 resultantly alleviate abrasions to the cervix. This condition is particularly undesirable in patients with severe tilting of the cervix.

A common conception cap 10 may be provided of a size suitable to fit a majority of women. In one application, a common conception cap 10 may have an inner diameter of approximately 33mm. Such an inner diameter may be suitable for parous and nuliparous women.

The dome 14 may extend from the annular rim 12 and define a receptacle area 26. The dome 14 may generally be in the shape of a thimble, and may include a closed tip 28, a base portion 30, and a sidewall 32 extending between the base portion 30 and the tip 28. As will be more appreciated below, the sidewall 32 may be collapsible for proper positioning relative to the cervical os. Such collapse of the sidewall may be particularly useful for treating women with a tilted cervix.

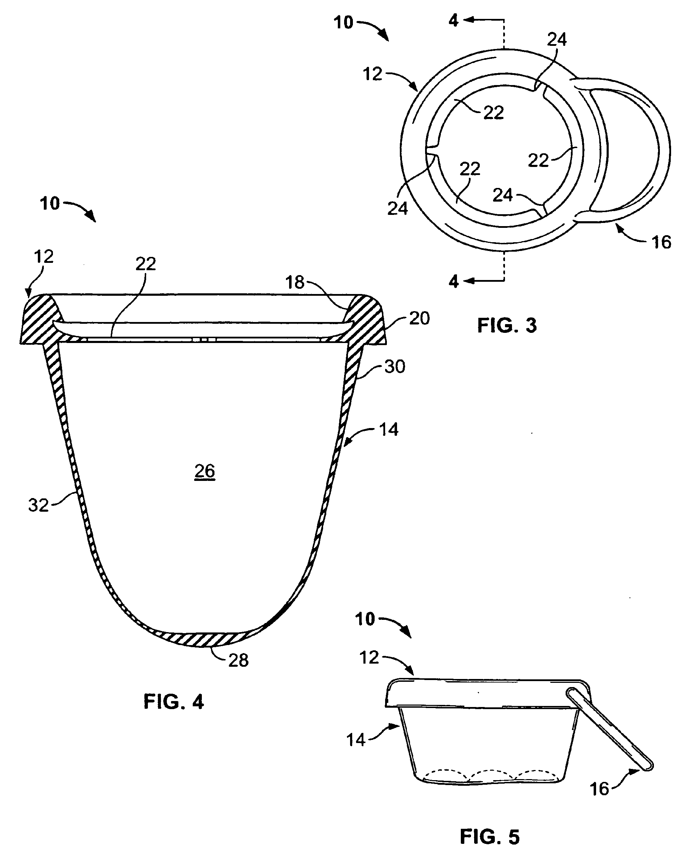

The dome 14 may be constructed of a thin, flexible material. Particular materials are addressed below. The dome 14 may be configured to facilitate a desired collapse of sidewall 32 which effectively creates a raised floor or tip of the conception cap 10. One such configuration is shown in Figure 5. In this manner, the contents of the receptacle area 26 is most effectively positioned relative to the cervical os, rather than a pinching of the tip that may undesirably preclude access to the cervical os.

The closed tip 28 may include a first thickness and the sidewall 32 may include a second thickness. The first thickness may be greater than the second thickness. In one particular application, the closed tip 28 may have a nominal thickness of approximate 0.889 (035 inches). In this particularly application, a portion of the sidewall 32 may have a nominal thickness of about 0.30 (.012 inches). This portion of the sidewall 32 may extend from proximate the closed tip 32 to proximate the base portion 30. A transition area may be defined approximately one-third the way from the annular rim 12 to the closed tip 28 that effectively defines the base portion 30 and has a third thickness. The third thickness may be greater than the second thickness and transition from a thickness of about 0.30 mm (.012 inches) to about (0.991 mm (.039 inches).

While the particular dimensions disclosed above have proven suitable for departing the desired collapse of the sidewall 32 during use, other dimensions may be employed within the scope of the present teachings. Particular dimensions will depend on material choices, among other factors. Important to this particular aspect of the present teachings, however, is that the thickness of the closed tip 28 be greater than the thickness of the sidewall 32.

The handle 16 may facilitate insertion and removal of the conception cap 10 and may be integrally-molded with the annular rim 12. The handle 16 may define a closed loop. The handle 16 may extend from the annular rim 12 at an angle to a plane defined by the annular rim 12. In one application, the handle 16 may extend from the annular arm 12 at an angle of approximately 45 degrees. It will be appreciated that the handle 16 may be oriented relative to the annular rim 12 at other angles within the scope of the present teachings. Preferably insofar as this particular aspect is concerned, the handle 16 is oriented at an angle of at least about 10 degrees and no greater than about 60 degrees.

It is contemplated that dome 14, annular rim 12, gripping flanges 22, and handle 16 will be made of a material suitable to use in the vagina. As used herein, the phrase "formed of a material suitable for use in the vagina" shall mean formed of a food grade or better material (*e.g*., food grade, medical grade, implantable grade, etc.). The cap 10 may be formed of a non-resilient flexible material, such as a silicone-based material. This material may or may not be formulated with biologically active components. These components may be released therefrom in an amount effective to achieve its purpose during use.

Types of silicone-based materials suitable for use herein are known in the art and include high-consistency and low-consistency silicone-based elastomers prepared using a variety of well-known methods (e.g., platinum-cured systems) selected for compatibility with biological tissue and particular active ingredients being released by the conception cap. An example of a biologically active agent that could be released by the cap is one that would alter pH, or effect semen activity.

The conception cap 10 may be incorporated into a kit such as that generally described in U.S. Patent No. 5,857,959. In addition to the various components described in U.S. Patent No. 5,857,959 the kit may include a lubricant and one or more practice caps. The lubricant may be a spermfriendly intimate moisturizer used to coat the interior of the vagina and the cervix. The practice caps may be shaped like the actual cap 10 and allow the user to be comfortable using the cap 10.

The conception cap 10 of the present invention can be used in a method of achieving conception in a mammalian subject. The method may generally include providing a conception cap 10 including a collapsible sidewall. The conception cap 10 is inserted into the vaginal cavity and positioned over the cervix. The conception cap 10 concentrates all available sperm at the opening of the cervical os. As such, the sperm is in contact with the cervical mucous and protected from the environment of the vaginal cavity.

Following sexual intercourse, the vaginal cavity relaxes, thereby causing compression of the conception cap 10 against and collapsing of the sidewall of the conception cap 10. This collapsing of the sidewall brings the closed tip of the cap 10 closer to an annular rim of the cap while providing a direct path for the sperm supported by the cap 10 to the cervical os. The pool of available sperm is placed in an optimum position relative to the cervix. This is of particular significance for a woman having a tilted cervix. If the woman's cervix is tilted (pointed in an abnormal direction), it may not come into contact with the semen pool. A tilted cervix may be the result of anatomy or adhesions that cause it to tilt from something like C-section surgery.

Sperm within the cap 10 has a much greater opportunity to meet an egg. The sperm do not have to deal with such issues as: making the long journey through the vaginal cavity to the cervix; being pulled out of the vaginal cavity by the penis; becoming lost in the vagina; being flushed from the vagina by gravity; being met by a hostile vaginal environment; or not pooling in the right location to contact the cervix.

While specific aspects of a particular embodiment have been described in the specification and illustrated in the drawings, it will be understood by those skilled in the art that various changes may be made and equivalence may be substituted for elements thereof without departing from the scope of the present teachings as defined in the claims. Furthermore, the mixing and matching of features, elements and/or functions may be expressly contemplated herein so that one skilled in the art would appreciate from the present teachings that features, elements and/or functions of one example may be incorporated into other examples as appropriate, unless described otherwise above. Moreover, many modifications may be made to adapt a particular situation or material to the present teachings without departing from the essential scope thereof. Therefore, it may be intended that the present teachings not be limited to the particular examples illustrated by the drawings and described in the specification as the best mode of presently contemplated for carrying out the present teachings but that the scope of the present disclosure will include any embodiments following within the forgoing description and the appended claims.

## Claims

1. A conception cap (10) for positioning over a cervix to concentrate semen and promote fertilization comprising:
- an annular rim (12); and
- a dome (14) extending from the annular rim (12), the dome (14) defining a receptacle area (26) and including a closed tip (28), a base portion (30) and a sidewall (32) extending between the closed tip (28) and the base portion (30), wherein
- the annular rim (12) and the dome (14) are formed of a material suitable for use in the vagina, the dome (14) having a first thickness at the closed tip (28) and a second thickness at the sidewall (32),
**characterized in that**
the first thickness is greater than the second thickness such that the sidewall (32) is collapsible in a manner to raise a floor defined by an inner surface of the closed tip (28) toward the annular rim (12).

2. The conception cap of claim 1, wherein the base portion (30) includes a third thickness, the third thickness being greater than the second thickness.

3. The conception cap of claim 1, wherein the conception cap (10) is adapted to be used with semen within the receptacle area (26) of the dome (14).

4. The conception cap of claim 1, further comprising a handle (16) integrally-molded to the annular rim (12).

5. The conception cap of claim 1, wherein the conception cap (10) is comprised of a silicone-based material.

## Patentansprüche

1. Inseminationskappe (10) zum Positionieren auf einer Zervix, um Samenflüssigkeit zu sammeln und eine Fertilisation zu fördern, umfassend:
- einen ringförmigen Rand (12) und
- eine Kuppe (14), die sich von dem ringförmigen Rand (12) erstreckt, wobei die Kuppe (14) einen Aufnahmebereich (26) festlegt und eine geschlossene Spitze (28), einen Basisabschnitt (30) und eine Seitenwand (32) umfasst, welche sich zwischen der geschlossenen Spitze (28) und dem Grundabschnitt (30) erstreckt, wobei
- der ringförmige Rand (12) und die Kuppe (14) aus einem Material gebildet sind, das sich für die Anwendung in der Vagina eignet, und die Kuppe (14) an der geschlossenen Spitze (28) eine erste Dicke und an der Seitenwand (32) eine zweite Dicke hat,
**dadurch gekennzeichnet, dass** die erste Dicke derart größer ist als die zweite Dicke, dass die Seitenwand (32) auf eine Weise zusammenfaltbar ist, um einen Boden, der durch eine innenliegende Fläche der geschlossenen Spitze (28) festgelegt ist, in Richtung zu dem ringförmigen Rand (12) anzuheben.

2. Inseminationskappe nach Anspruch 1, wobei der Basisabschnitt (30) eine dritte Dicke umfasst, wobei die dritte Dicke größer ist als die zweite Dicke.

3. Inseminationskappe nach Anspruch 1, wobei die Inseminationskappe (10) geeignet ist, in dem Aufnahmebereich (26) der Kuppe (14) mit Samenflüssigkeit verwendet zu werden.

4. Inseminationskappe nach Anspruch 1, ferner umfassend einen Griff (16), der einstückig an dem ringförmigen Rand (12) angeformt ist.

5. Inseminationskappe nach Anspruch 1, wobei die Inseminationskappe (10) aus einem Material auf Basis von Silikon besteht.

## Revendications

1. Cape de conception (10) pour positionner sur un col de l'utérus afin de concentrer de sperme et promouvoir une fertilisation, comprenant :
- un rebord annulaire (12) ; et
- un dôme (14) s'étendant du rebord annulaire (12), le dôme (14) définissant une zone collectrice (26) et incluant une pointe fermée (28), une portion de base (30) et une paroi latérale (32) s'étendant entre la pointe fermée (28) et la portion de base (30), dans laquelle
- le rebord annulaire (12) et le dôme (14) sont formés d'un matériau approprié à une utilisation dans le vagin, le dôme (14) ayant une première épaisseur au niveau de la pointe fermée (28) et une deuxième épaisseur au niveau de la paroi latérale (32),
**caractérisée en ce que** la première épaisseur est supérieure à la deuxième épaisseur de sorte que la paroi latérale (32) est repliable de manière à soulever un fond défini par une surface interne de la pointe fermée (28) vers le rebord annulaire (12).

2. Cape de conception selon la revendication 1, dans laquelle la portion de base (30) inclut une troisième épaisseur, la troisième épaisseur étant supérieure à la deuxième épaisseur.

3. Cape de conception selon la revendication 1, dans laquelle la cape de conception (10) est adaptée à être utilisée avec du sperme au sein de la zone collectrice (26) du dôme (14).

4. Cape de conception selon la revendication 1, comprenant en outre une poignée (16) moulée de manière intégrale au rebord annulaire (12).

5. Cape de conception selon la revendication 1, dans laquelle la cape de conception (10) est constituée d'un matériau à base de silicone.
